(19) **Europäisches Patentamt European Patent Office Office européen des brevets**

(11) **EP 2 586 323 A1**

(12) **EUROPEAN PATENT APPLICATION**
published in accordance with Art. 153(4) EPC

(43) Date of publication:
01.05.2013 Bulletin 2013/18

(21) Application number: 11797646.4

(22) Date of filing: 28.03.2011

(51) Int Cl.:
*A43D 1/02* (2006.01)   *A61B 5/107* (2006.01)
*G01B 11/02* (2006.01)

(86) International application number:
**PCT/ES2011/070207**

(87) International publication number:
**WO 2011/161285 (29.12.2011 Gazette 2011/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 25.06.2010 ES 201030981

(71) Applicant: **Alu Group, S.L.**
**03203 Elche (Alicante) (ES)**

(72) Inventors:
• **HERNÁNDEZ STARK, Rafael**
**E-03203 Elche (Alicante) (ES)**

• **MARTÍNEZ IBÁÑEZ, Pascual**
**E-03203 Elche (Alicante) (ES)**
• **MONTIEL PARREÑO, Enrique**
**E-03203 Elche (Alicante) (ES)**
• **SABATELL HERRERO, Iván**
**E-03203 Elche (Alicante) (ES)**

(74) Representative: **Ungria López, Javier**
**Avda. Ramón y Cajal, 78**
**28043 Madrid (ES)**

(54) **FOOT-MEASURING METHOD AND DEVICE**

(57)    The invention relates to a device that comprises: an element (1) for placement of a foot, which is provided with means (2-4, 7, 8) for positioning the foot (13) of a user and a mark (6) corresponding to a true measurement on the element (1); a camera (11) for capturing an image of the element (1) with the foot (13) placed in the required position; and a processor (12) for processing the image obtained in accordance with a conventional augmented reality program (AR), in order to obtain the true measurement of the length of the foot and to determine a size in accordance with conventional established sizes. The invention facilitates the purchase of footwear, both in a store and online.

FIG. 3

EP 2 586 323 A1

## Description

## OBJET OF THE INVENTION

The present invention, as expressed in the wording of this specification, relates to a method and a device whose object is to determine the measurement of the foot in order to obtain the appropriate footwear size for the measured foot.

Another object of the invention is to allow an user to carry out the measurement of the foot from a remote location, as it can be for instance, from its own house, so as make remote shopping, like for instance could be the case of buying from a catalogue and/or on the internet.

## BACKGROUND OF THE INVENTION

The disadvantages of the buying process of footwear in a physical shop are well known, given the need of trying on more than one footwear size before finding the appropriate for the user. This same fact becomes a tangible and significant handicap when buying on a virtual shop, by means of internet, given that in this case the user does not have the possibility of trying on the footwear.

To promote the buys online of footwear, it is necessary to offer to the client some security in the fact that the footwear that he/she is buying suits his/her foot.

In the state of the art, it is known the existing correlation between the length of the foot and the size of the footwear in each of the different shoe-size systems implemented in different countries (European sizes, British sizes, USA sizes). That is why, a device that allows obtaining the length of the foot can provide by direct relation and by means of a table of equivalences, the shoe size number corresponding to the foot of each user. A device of these characteristics facilitates the shopping in a physical store, given that it minimizes the time spent trying on the footwear and it also may prove of invaluable help especially in the case of children, whose foot experiments a fast growth and because of their age they are less prone to provide precise data about the fit.

On this point it should be noted that in the state of the art, it is known the use of augmented reality processes consisting in an application running in a processor so that on a true image a variable amount of additional information is added, generally by superimposition, in the form of virtual elements, which can be constituted by text, portions of bi-dimensional images, fix as well as in movement, or tridimensional objects. Thus, the image captured by a video camera is treated by the computer application, which enlarges it together with the virtual objects and the information that comes with them, in order to subsequently display said enlarged image on a screen, being the true image and the virtual elements associated to the same coordinate system that allows handling and displaying them together and in an integrated manner.

In spite of the fact that there are numerous patented devices wherein their ability to measure feet is specified, some of them, significantly old, as it is the case for instance of the patent documents GB 191494614 and US 1529167, the truth is that none of them uses the superposition of a virtual element on a true image of a foot, by means of the distinctive techniques of the augmented reality (AR), in order to find out the foot measures.

In the state of the art, it is possible to find devices and/or methods based on the use of measuring apparatus having fix or movable physical elements, as the ones described in the patent document GB 5901954 A, JP 2008206916, WO 0200608896 or ES 250287Y. There are also devices based on systems of light sensors, as the ones described in the patents WO 200987618 or US 2007253004.

Other foot-measuring devices are based on the scanning of the foot in two or three dimensions, as described in the patent document WO 2004034832; on a three-dimensional reconstruction from bi-dimensional images, as described in the patent document GB 2417153, or US 2006104503; on the measurement of photos with superposed or known scales, as described in the patent document KR 20060109669; on paper or foam printings, as described in the patent document WO 0135047; or on mathematical projections by using descriptive matrix, as described for instance in the patent document GB 2352048.

Moreover, it should be highlighted that no relation between AR and the measurement concept has been found, beyond vague references in the measurement of geographical points of interest and the correlation of the measurements with true reference points, as described for instance in the patent document JP 2007156561; the evaluation of distances for the correlation between true and virtual in reference and surgical navigation systems with AR, as described for instance in the patent document US 2005215879; or true-time measuring systems on referenced images in the scope of the ophthalmic exam of the back of the eye, as described for instance in the patent document US 5912720A.

Summarizing, the applicant does not have any knowledge about the existence of a method and/or device that through the use of AR allows measuring a foot and therefore to find out the according footwear size number.

## DESCRIPTION OF THE INVENTION

The invention provides a method and a device based on the use of augmented reality (AR), by means of which the length of the foot, and optionally the width thereof or whatever other measures may be necessary can be found out, by superimposing a movable virtual element onto a true image of the users foot positioned on a sole.

Thus, the invention provides a new foot-measuring method, which comprises a stage in which an element for the placement of a foot is obtained, which is provided with means for positioning the foot and a mark, said mark corresponds to a true measurement on the sole, as for instance can be the distance from the rear edge of the sole to the mark, and in such a way that said mark is provided in a position on the element for the placement of the foot, wherein it is visible after placing the foot thereon, in the position required by the positioning means.

Next, an image of the element for the placement of a foot is obtained, with the foot placed in the required position and the obtained image of the element and the foot is processed in a processor, by means of a conventional procedure of augmented reality to superimpose at least one virtual object on the mark of the processed image, displaying the obtained result, that is to say, the processed image in which the virtual object is superimposed on the mark.

Thereafter, the procedure of the invention foresees obtaining the virtual value of the true measurement in the processed image, in such a way that when moving the virtual object up to the point in which the front part of the foot ends, it allows calculating the distance travelled according to the movement of the virtual object on said processed image, and also allows obtaining the virtual length of the foot in the image out from the distance travelled by the virtual object and from the virtual value that has the true measurement determined in the processed image, in such a way that after obtaining the virtual length of the foot, this value is transformed into the true value of the foot length, out from the true measurement included in the sole, determined by the mark, which is known by the processor.

To obtain the virtual length of the foot in the image, the vertical projection of the heel of the foot corresponding to the rearmost point of the foot is obtained, which is carried out using the means for positioning the foot that are provided in the element for the placement of a foot. Next, a central line, longitudinal to the foot is traced and a line perpendicular to the longitudinal central line and tangent to the point where the front part of the foot ends is traced. Then, the virtual object is placed in correspondence with this last line, in order to obtain the virtual length of the foot as described above.

Based on the measurement of the true length of the foot, the size of said foot is obtained, accessing to a table of equivalences stored in the processor, according to the sizes established in different countries, in such a way that the user knows his/her shoe-size in any of the systems adopted by each country.

In the preferred embodiment of the invention the element for the placement of a foot is delimited by a sole, whose obtaining stage is carried out by printing thereof on a sheet of paper, in which the means for positioning the foot are determined by cutting lines, folding lines and positioning lines, which enables after realizing the cut along said cutting lines, and folding along the according folding lines, to obtain two wings joined together by an intermediate section, which are arranged perpendicular to the sole surface, placing the wings according to the established positioning lines, and next said wings are pasted onto the surface of the sole, so as a result the intermediate section and the wings establish a limit which constitutes the means for positioning the foot. The intermediate section constitutes the means that allows obtaining the vertical projection of the foot heel, to realize the measurement of the virtual length in the obtained image. In the preferred embodiment said intermediate section is located in correspondence with the rear edge of the sole.

As it has been described, the foot size can be obtained after obtaining the true measurement of the foot length, but additionally and according to the described procedure, the procedure of the invention also allows obtaining the true value of the width of the foot, the length of the arch or any other required measurement, to obtain the foot size based on these obtained measures, achieving a greater accuracy when obtaining the foot size of the user and/or a more ergonomic footwear adapted to the conditions of the user's foot, having access to the according table of equivalences, similarly to the procedure previously described whereby only the length of the foot was being measured.

Moreover, the invention relates to a device which allows executing the different stages of the method described previously, to that end said device comprises an element for the placement of a foot, which in the preferred embodiment of the invention is delimited by a sole having the characteristics already described in the procedure of the invention. It is foreseen that the element for the placement of a foot can be a sock provided with a mark corresponding to a true measurement. Furthermore, the device comprises a camera for capturing images of the sole with the foot placed in the required position by the means for positioning the foot provided in the sole, in such a way that the image captured by the camera is processed in a processor, in accordance with an augmented reality program to carry out the measurements as described in the method of the invention.

In an embodiment of the invention it is provided that the sole can incorporate printed silhouettes of feet with different sizes, to facilitate the placement of the foot of the user.

Therefore, based on the disclosed description, the invention allows obtaining the size of the foot of an user, in a fast, easy and economical way, and in such a way that it is considerably easier to buy footwear in a physical store, by avoiding the user the need of trying on different footwear and at the same time it promotes the purchase of footwear online.

Next, for better comprehension purposes of this specification, and making an integral part thereof, a series of figures are attached, in which by way of non limiting illustration the object of the invention has been represented

## BRIEF DESCRIPTION OF THE FIGURES

**Figure 1.-** Shows in a schematic form a possible embodiment of the element for the placement of a foot, which is constituted by a sole that is obtained by printing it on a sheet of paper.

**Figure 2.-** Shows a perspective view, equivalent to the previous figure, wherein the means for positioning the foot have been obtained after making cuts and folds on the sheet of paper of the previous figure.

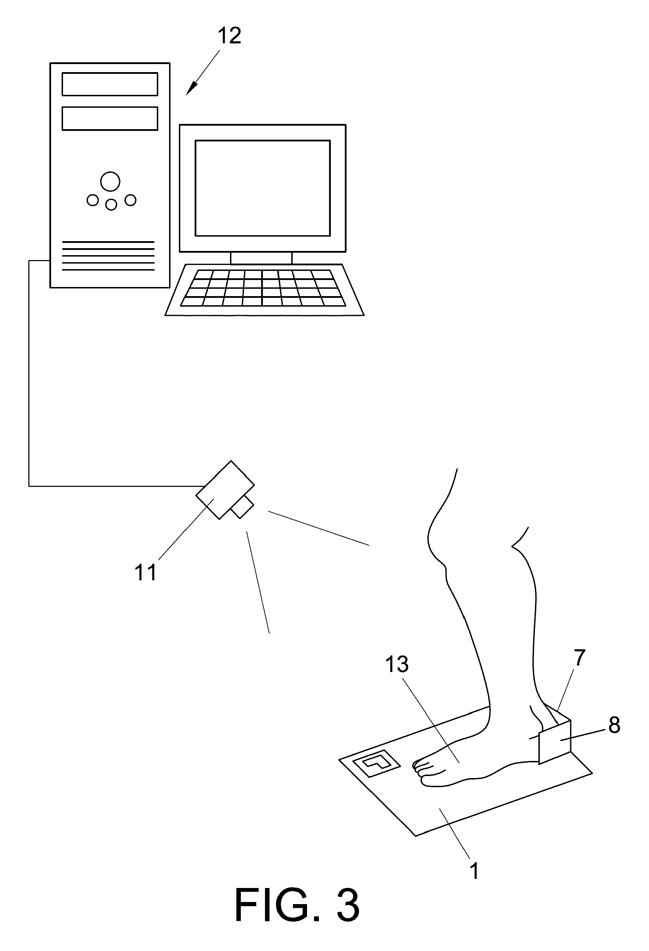

**Figure 3.-** Shows a schematic representation of the device of the invention and the stage of capturing the image of the foot of the user correctly placed on the sole.

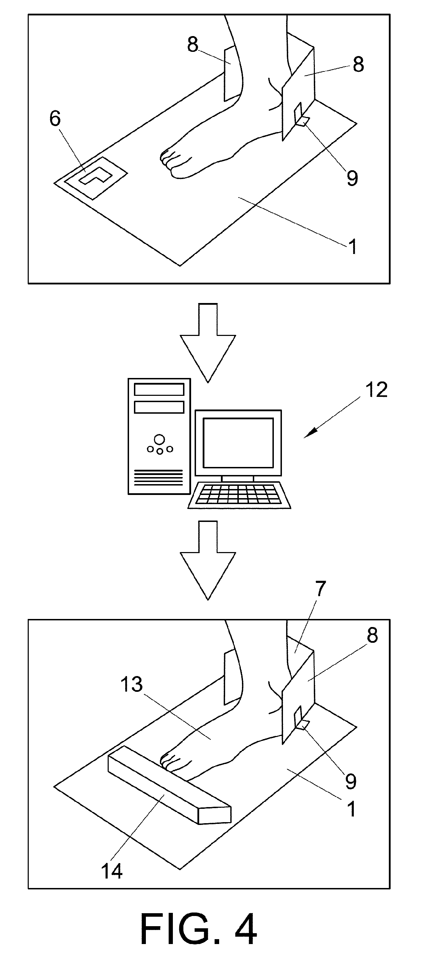

**Figure 4.-** Shows a schematic representation of an example of the true image with the virtual element used to measure the length of the foot, that is to say, it shows an augmented reality image as obtained by the processor.

## DESCRIPTION OF THE PREFERRED EMBODIMENT

Next, a description of the invention based on the aforementioned figures is made.

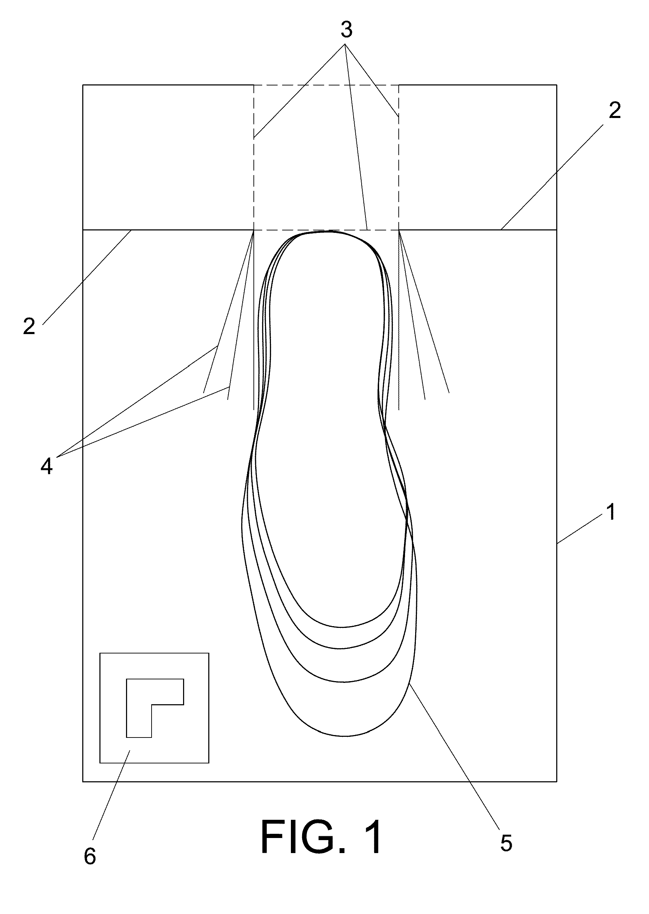

In view of figure 1, it can be seen how the device of the invention includes an element 1 for the placement of a foot, which in the exemplary embodiment is constituted by a sole 1, printed on a sheet of paper, which is provided with cutting lines 2, folding lines 3 and positioning lines 4, in such a way that by cutting along the lines 2 and folding along the lines 3, two wings 8 are obtained, which are linked together by an intermediate section 7, so the wings 8 are placed on the positioning lines 4 and are fixed by means of an adhesive tape 9 onto the surface of the sole 1, thus obtaining a sole with means for positioning the foot 13 on which the heel has to be placed, in such a way that its rear part abuts against the intermediate section 7 and the foot sides against the wings 8.

Also, the sole 1 is provided with printed silhouettes 5 of feet with different sizes to facilitate the placement of the foot 13.

Moreover, it should be highlighted that on the surface of the sole 1 a mark 6 has been incorporated, which determines a true distance from the rear part of the sole 1 to the position held by the mark 6 therein.

Furthermore, the device of the invention is provided with a camera 11 for capturing images, which is connected to a processor 12 that includes a computer program of augmented reality, (AR). The processor is constituted by a personal computer 12, and the camera 11 can be external or be integrated in the personal computer 12 itself, as is the case with many portable laptops from the state of the art.

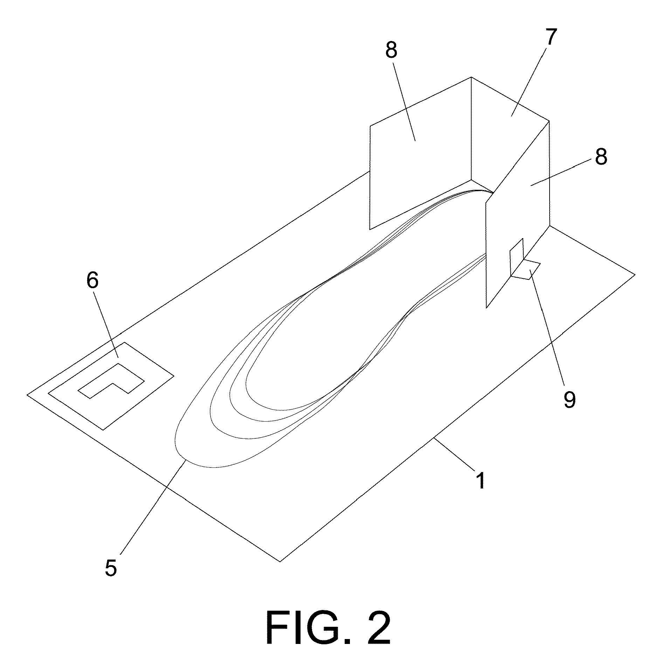

Thus, based on the description made, it should be understood that once the sole has been obtained, according to what has been represented in figure 2, the foot 13 of an user can be positioned so the rear part of its heel comes into contact with the intermediate section 7, and the sides of said heel with the wings 8, and all that guided by the printed silhouettes 5. In this position, in which the foot is correctly placed, as established by the positioning means, delimited by the wings 8 and the intermediate section 7, an image of the sole 1 is obtained, with the foot 2 placed in the required position, in such a way that the obtained image is processed by the personal computer 12 according to the computer program of augmented reality, through which one or more virtual elements are added to the true captured image, and on the other hand the captured image is analyzed, while allowing to interact with the user and determining the foot measurements as described below.

When the processing of the obtained image of the sole and the foot is realized through the procedure of augmented reality, a virtual object 14 is superimposed on the mark 6, in such a way that it enables the computer program to establish the same coordinate and spatial location system for the captured true image as for the virtual element 14 that is superimposed onto the captured true image when executing the augmented reality program. From that moment on the user can move back and forth the virtual element 14 by actuating the controls provided to that effect in the keyboard and/or mouse.

Moreover, the vertical projection of the foot heel corresponding to the rearmost point of the foot is obtained, which is delimited by the intermediate section 7, and a central line, longitudinal to the foot is traced. Next, a line perpendicular to the central longitudinal line and tangent to the point where the front part of the foot 13 ends, is traced, and the virtual object 14 is moved until placing it visually in correspondence with the line perpendicular to the central longitudinal line and tangent to the point where the front part of the foot (13) ends, which corresponds to the front position more distal of the foot of the user, that is to say, normally in correspondence with the front extremity of the big toe thereof.

In this moment the computer program calculates the length from the vertical projection of the heel of the foot to the front part of the foot, based on the ratio between both aforementioned coordinate systems, true and virtual, which is known and has been established beforehand.

To this end a *priori* the true distance from the rear part of the sole printed on the paper to the position held by the mark 6 therein, is known, as it has been described before. Also, the virtual distance existing between the rear part of the paper and the place where the mark 6 is detected is known, in the internal units that are appropriate for the computer program and that belong to the virtual coordinate system. From this data, the travel that takes place on said virtual coordinate system every time that a control on the keyboard or the mouse is actuated upon, is calculated.

Once this data is known, all there is to do is adding, when the travel is carried out towards the tip of the foot or subtracting, when the travel is carried out towards the heel of the foot, the travel that the user has caused on the virtual element 14 to calculate the final value of the virtual length of the foot of the processed image.

From that moment on, by means of a simple rule of three the correspondence of said virtual length with regard to the true length of the foot 13 is established. If the virtual length from the limit determined by the intermediate section 7 to the mark 6 ($LT_V$) is equal to the true length of the limit 7 preceding the mark 6, then the true length of the foot ($LP_r$) is equal to the virtual length of the foot ($LP_V$) multiplied by the true length from the limit behind the printed mark, the result being divided by the virtual length from the limit 7 to the printed part 6, that is to say:

$$LP_R = \frac{LP_V \times LT_R}{LT_V}$$

Therefore the true length of the foot 13 is obtained, whose value can be related to a size of any of the footwear sizing systems used nowadays in different countries, by means of a table of equivalences of foot length/size that can be stored in the computer 12.

**Claims**

1. **Foot-measuring method, characterized in that** it comprises the following stages:

   - obtaining an element (1) for the placement of a foot (13) which is provided with means (2-4, 7, 8) for positioning the foot (13) and a mark (6), corresponding to a true measurement on the element (1), which is provided in a position in which it remains visible after placing the foot (13) on said element (1) in the position required by the positioning means (7, 8);
   - obtaining an image of the element (1) with the foot (13) placed in the required position;
   - processing the obtained image of the element (1) and foot (13) in a processor (12) by means of a conventional procedure of augmented reality (AR); for
   - superimposing at least one virtual object (14) on the mark (6) of the processed image, and displaying the processed image together with the virtual objective (14);
   - obtaining the virtual value of the true measurement in the processed image;
   - moving the virtual object (14) to the point where the front part of the foot (13) ends;
   - calculating the distance travelled by the virtual object moved on the processed image; and

   - obtaining the virtual length of the foot (13) in the image based on the distance travelled by the virtual object (14) and the virtual value of the true measurement in the processed image;
   - transforming the virtual length of the foot obtained into the true length of the foot, based on the true measurement included in the element (1), known by the processor (12).

2. **Foot-measuring method,** according to claim 1, **characterized in that** obtaining the virtual length of the foot (13) in the image based on the distance travelled by the virtual object (14) and the virtual value of the true measurement in the processed image; it further comprises obtaining the vertical projection of the heel of the foot, corresponding to the rearmost point of the foot, by the intermediary of the means (7) for positioning the foot; tracing a central line longitudinal to the foot; tracing a line perpendicular to the central line longitudinal and tangent to the point where the front part of the foot (13) ends, and placing the virtual object in correspondence with the line perpendicular to the longitudinal central line and tangent to the point where the front part of the foot (13) ends.

3. **Foot-measuring method,** according to claim 1, **characterized in that** it comprises a stage for obtaining a size of the foot (13), based on the measurement of the true length of the foot, obtained by accessing to a table of equivalences stored in the processor (12).

4. **Foot-measuring method,** according to claim 1, **characterized in that** the stage for obtaining the element (1) comprises the printing of a sole (1) on a sheet of paper, wherein the means for positioning the foot are determined by cutting lines (2), folding lines (3) and positioning lines (4), so after cutting and folding along the according cutting (2) and folding lines (3), two wings (8) are obtained, which are joined together by an intermediary section (7), perpendicular to the sole (1) surface, and then, positioning the wings (8) in accordance with the positioning lines (4) in the indicated position and pasting them onto the surface of the sole (1), so the intermediate section (7) and the wings (8) define a limit, which constitutes the means for positioning the foot (13).

5. **Foot-measuring method,** according to claim 1, **characterized in that** it comprises a stage for obtaining the true value of the measurement of one parameter of the foot (13) selected from the width, arch length and a combination thereof; and a stage for obtaining a size of the foot based on the true measurement of the foot (13) length and the true measurement selected from among the width, the arch length and a combination thereof, accessing to a table of equivalences stored in the processor (12).

6. **Foot-measuring device, characterized in that** it comprises an element (1) for the placement of a foot (13), which is provided with means for positioning the foot of the user and a mark (6), corresponding to a true measurement on the element (1), which is provided in a position such that it remains visible after placing the foot (13) on the element (1) in the position required by the positioning means; a camera (11) for capturing an image of the element (1) with the foot (13) placed in the required position, an a processor (12) for processing the image of the element (1) and the foot (13) obtained, in accordance with a conventional augmented reality program (AR), in order to obtain the true measurement of a parameter of the foot selected from length, width, arch length and a combination thereof.

7. **Foot-measuring device,** according to claim 6, **characterized in that** the element (1) is constituted by a sole (1) in which the means for positioning the foot of the user, are determined by a sheet (7) perpendicular to the surface of the sole (1) to define a limit for the rear part of the heel of the foot (13), which constitutes the rearmost point of the foot (13).

8. **Foot-measuring device,** according to claim 7, **characterized in that** the sheet perpendicular to the surface of the sole (1), comprises two wings (8) joined together by an intermediate section (7), in order to define a limit for the rear and lateral parts of the heel of the foot (13).

9. **Foot-measuring device,** according to claim 6, **characterized in that** the sole (1) is provided with printed silhouettes (5) of a foot with different measures in order to facilitate the foot positioning (13).

FIG. 1

FIG. 2

FIG. 3

# FIG. 4

INTERNATIONAL SEARCH REPORT

| | International application No. |
|---|---|
| | PCT/ES2011/070207 |

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
A43D, A61B, G01B

Documentation searched other than minimum documentation to the extent that such documents are included in the fields searched

Electronic data base consulted during the international search (name of data base and, where practicable, search terms used)

EPODOC, INVENES, WPI

C. DOCUMENTS CONSIDERED TO BE RELEVANT

| Category* | Citation of document, with indication, where appropriate, of the relevant passages | Relevant to claim No. |
|---|---|---|
| Y | US 6289107 B1 (NIKE INC.) 11/09/2001, Abstract, figure 2, 6, 8G, 10A, 10E, 12B; column 1, line 56 - column 2, line 11; column 3, line 36 - column 4, line 2; column 7, line 53 - column 9, line 9; column 12, line 19 - column 12, line 36; column 13, line 54 - column 14, line 2; column 15, line 10 - line 59 | 1-9 |
| Y | US 2006008779 A1 (SHAND ANNE-MARIE ET AL.) 12/01/2006, figure 8, paragraphs[0065 - 0073]; | 1-9 |
| Y | US 2005061332 A1 (GREENAWALT KENT S ET AL.) 24/03/2005, paragraphs[0002 - 0012]; paragraphs[0020 - 0048]; Todas las figuras. | 1-9 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 19/07/2011 | **(05/08/2011)** |
| Name and mailing address of the ISA/ OFICINA ESPAÑOLA DE PATENTES Y MARCAS Paseo de la Castellana, 75 - 28071 Madrid (España) Facsimile No.: 91 349 53 04 | Authorized officer M. Rivas Sáiz Telephone No. 91 3498595 |

Form PCT/ISA/210 (second sheet) (July 2009)

**INTERNATIONAL SEARCH REPORT**

| International application No. |
| --- |
| PCT/ES2011/070207 |

| C (continuation). | DOCUMENTS CONSIDERED TO BE RELEVANT | |
| --- | --- | --- |
| Category * | Citation of documents, with indication, where appropriate, of the relevant passages | Relevant to claim No. |
| Y | US 2009190808 A1 (CLAUS MICHAEL J ) 30/07/2009, Abstract; paragraphs[0005 - 0008]; figure 4, | |
| A | JP 2008206916 A (OSHIMA SHOJI KK  ) 11/09/2008, Todas las figures | 1-9 |
| A | DE 202009000809 U1 (KINZEL DIETRICH ) 26/03/2009, figures 1 - 2. | 1-2 |

Form PCT/ISA/210 (continuation of second sheet) (July 2009)

## INTERNATIONAL SEARCH REPORT

Information on patent family members

International application No.

PCT/ES2011/070207

| Patent document cited in the search report | Publication date | Patent family member(s) | Publication date |
|---|---|---|---|
| US6289107 B | 11.09.2001 | NONE | |
| US2006008779 A | 12.01.2006 | CA2511274 A<br>GB2415878 A<br>AU2005202910 A | 02.01.2006<br>04.01.2006<br>02.02.2006 |
| US2005061332 A | 24.03.2005 | NONE | |
| US2009190808 A | 30.07.2009 | CA2713487 A<br>AU2009209231 A<br>WO2009097305 A<br>EP2245597 A<br>EP20090705019 | 06.08.2009<br>06.08.2009<br>06.08.2009<br>03.11.2010<br>28.01.2009 |
| JP2008206916 A | 11.09.2008 | NONE | |
| DE202009000809 U | 26.03.2009 | NONE | |

Form PCT/ISA/210 (patent family annex) (July 2009)

## INTERNATIONAL SEARCH REPORT

International application No.

PCT/ES2011/070207

CLASSIFICATION OF SUBJECT MATTER

*A43D1/02* (2006.01)
*A61B5/107* (2006.01)
*G01B11/02* (2006.01)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 191494614 A
- US 1529167 A
- GB 5901954 A
- JP 2008206916 B
- WO 0200608896 A
- ES 250287 Y
- WO 200987618 A
- US 2007253004 A
- WO 2004034832 A

- GB 2417153 A
- US 2006104503 A
- KR 20060109669
- WO 0135047 A
- GB 2352048 A
- JP 2007156561 B
- US 2005215879 A
- US 5912720 A